## Europäisches Patentamt

## European Patent Office

## Office européen des brevets

(19)

(11) Veröffentlichungsnummer: **0 100 296**
**A1**

(12) # EUROPÄISCHE PATENTANMELDUNG

(21) Anmeldenummer: **83810246.5**

(22) Anmeldetag: **08.06.83**

(51) Int. Cl.³: **A 61 M 5/14**

(30) Priorität: **14.06.82 CH 3668/82**
**02.07.82 CH 4051/82**

(43) Veröffentlichungstag der Anmeldung: **08.02.84**
**Patentblatt 84/6**

(84) Benannte Vertragsstaaten: **AT BE CH DE FR GB IT LI NL SE**

(71) Anmelder: **CIBA-GEIGY AG, Patentabteilung Postfach, CH-4002 Basel (CH)**

(72) Erfinder: **Geller, Leo, Dr., Rudolf Wackernagel-Strasse 14, CH-4125 Riehen (CH)**

(54) **Wirkstoffabgabevorrichtung für parenterales Applikationssystem.**

(57) Der aus zwei Halbschalen (11) und (12) zusammengesetzte Kunststoff-Behälter (10) ist durch eine gerippte Aluminiumfolie (13) in zwei Kammern (14) und (15) unterteilt und besitzt oben einen Einlass (18) und unten einen Auslass (19). Die beiden Kammern (14) und (15) weisen in ihrem obersten Teil je eine Entlüftungsöffnung (21) bzw. (22) auf. In der einen Kammer (14) befinden sich drei Überläufe (25a, 25b, 26), die in den Auslass (19) bzw. in die andere Kammer (15) münden. In letzterer befindet sich ein weiterer Überlauf (27), der in den Auslass (19) mündet, sowie ein plattenförmiger Träger (30) mit dem abzugebenden Wirkstoff. Die Flüssigkeit gelangt vom Einlass (18) über die eine Kammer (14) und den Überläufen (25a, 25b) in die andere Kammer (15), strömt dort von unten nach oben am Wirkstoffträger (30) vorbei und gelangt schliesslich über den Überlauf (27) zum Auslass. In einem Nebenpfad strömt Flüssigkeit direkt vom Einlass (18) über die Kammer (14) und den Überlauf (26) zum Auslass (19).

Die Vorrichtung ermöglicht eine sehr gleichmässige Wirkstoffabgabe und ist einfach und entsprechend wenig aufwendig herstellbar.

EP 0 100 296 A1

ACTORUM AG

- 1 -

CIBA-GEIGY AG                                    47-13959/K

Basel (Schweiz)


Wirkstoffabgabevorrichtung für parenterales Applikationssystem

Die Erfindung betrifft eine Wirkstoffabgabevorrichtung zum Einfügen in die Flüssigkeitsleitung eines parenteralen Applikationssystems gemäss dem Oberbegriff des Patentanspruchs 1.

Die intravenöse Verabreichung von Flüssigkeiten wie Blut, Blutersatz und anderen Infusionslösungen ist ein wichtiger Bestandteil der ärztlichen Behandlung von Patienten. In vielen Fällen wird ausserdem gleichzeitig bzw. zusammen mit der Infusionslösung auch noch irgendein anderes Medikament intravenös verabreicht, wofür eine Reihe von verschiedenen Systemen existieren. So ist z.B. bei einem aus der prioritätsälteren DE-A 32 28 595 (entsprechend den US-Patentanmeldungen Ser. Nos. 289082, 312491, 310047 und 325206) bekannten System dieser Art in die Infusionsleitung oder parallel zu einem Abschnitt derselben ein Behälter eingeschleift, in welchem der zu verabreichende Wirkstoff in einer besonderen Formulierung (Osmose-System) enthalten ist, welche eine über einen längeren Zeitraum andauernde Wirkstoffabgabe an die Infusionslösung gewährleistet. Der Nachteil dieses Systems besteht in erster Linie darin, dass der Wirkstoff unbedingt in einer speziellen Formulierung vorliegen muss und dass ausserdem das Einschleifen des Behälters in die Infusionsleitung, insbesondere wenn dies mittels einer Bypass-Leitung erfolgen soll, relativ umständlich ist. Ein weiterer Nachteil des bekannten Systems besteht darin, dass es relativ schwierig ist, allenfalls im Behälter vorhandene Luft aus diesem zu entfernen.

Durch die vorliegende Erfindung soll nun eine Wirkstoffabgabevorrichtung der eingangs definierten Art dahingehend verbessert werden,

- 2 -

dass sie einerseits eine möglichst gute, gleichmässige und kontrollierte
Abgabe des Wirkstoffs gewährleistet, wobei der Wirkstoff nicht in einer speziellen Formulierung vorzuliegen braucht, und dass sie anderseits konstruktiv einfach sowie einfach und leicht in der Handhabung
und Bedienung ist und ferner auch keine Schwierigkeiten mit der
Entlüftung auftreten.

Die erfindungsgemässe Wirkstoffabgabevorrichtung ist im Patentanspruch
1 beschrieben. Vorteilhafte Weiterbildungen ergeben sich aus den
abhängigen Ansprüchen.

Im folgenden wird die Erfindung anhand der Zeichnung näher erläutert.
Es zeigen:

Fig. 1          eine schematische Ansicht eines mit einer erfindungsgemässen Wirkstoffabgabevorrichtung ausgestatteten
                parenteralen Applikationssystems und

Fig. 2-6        eine Ansicht eines und einen Querschnitt sowie drei Längsschnitte durch ein Ausführungsbeispiel der erfindungsgemässen Wirkstoffabgabevorrichtung.

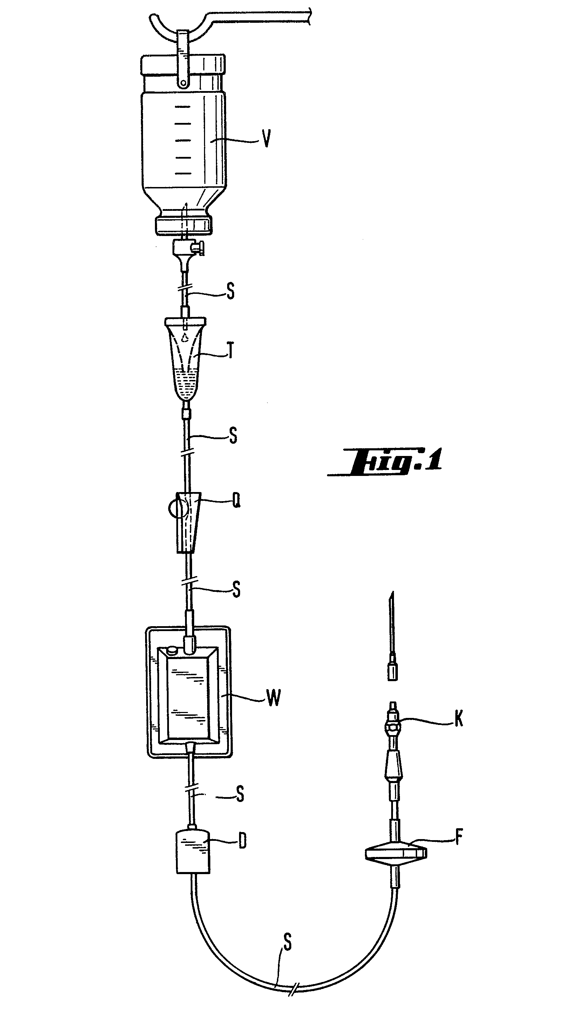

Das in der Fig. 1 rein beispielsweise dargestellte parenterale Applikationssystem umfasst typischerweise ein Vorratsbehältnis V für
eine zu verabreichende Flüssigkeit, beispielsweise physiologische
Kochsalzlösung oder dgl., eine Tropfeinrichtung T, eine Quetsche Q,
eine Wirkstoffabgabevorrichtung W, einen Durchflussbegrenzer D,
einen Sterilfilter F, eine Kanüle K und eine alle diese Teile verbindende Schlauchleitung S. Die spezifische Ausbildung der einzelnen
Bestandteile des Systems ist, mit Ausnahme der der Wirkstoffabgabevorrichtung W, für das Verständnis der vorliegenden Erfindung bedeutungslos und wird daher nicht näher beschrieben.

Soweit entspricht das System im wesentlichen dem bekannten Stand der
Technik, wie er beispielsweise durch die genannte DE-A-32 28 595

- 3 -

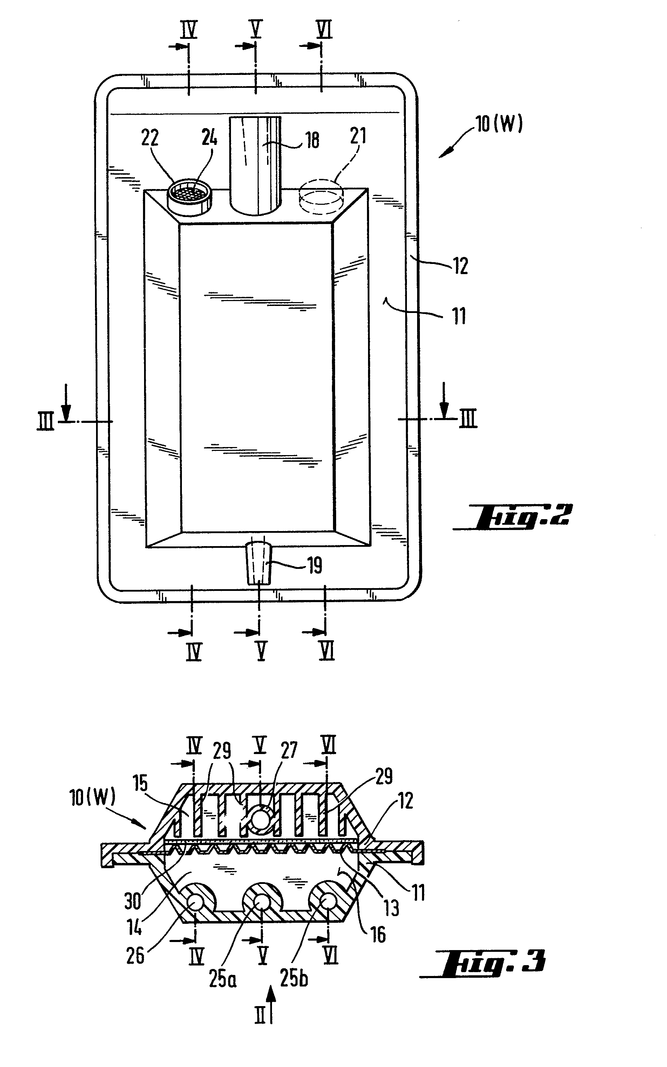

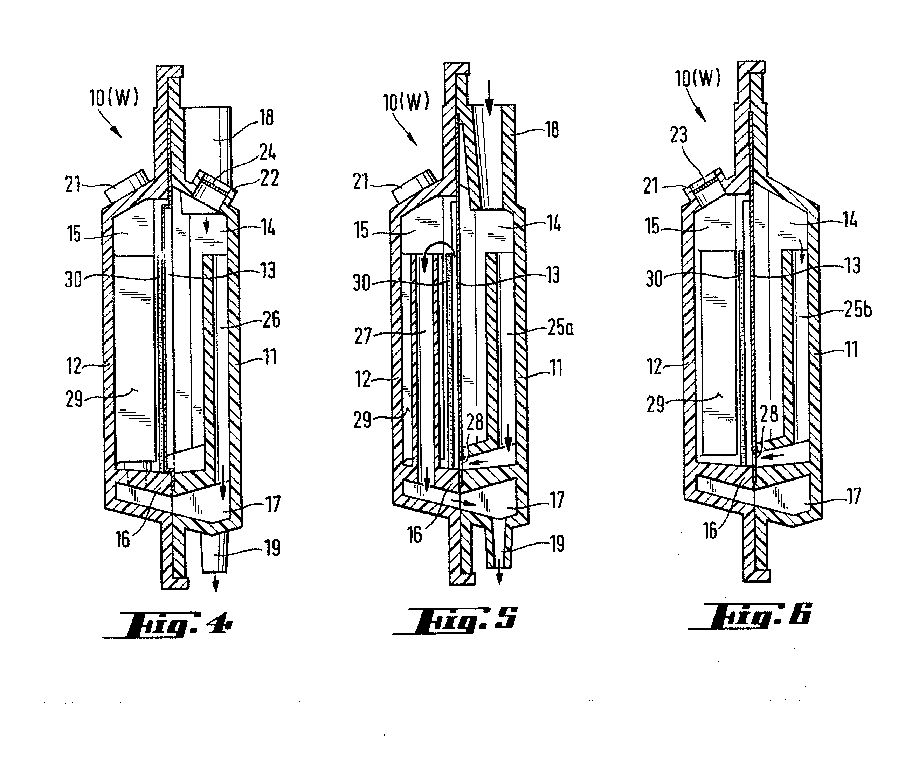

gegeben ist. Die Unterschiede zu diesem liegen lediglich in der Ausbildung der Wirkstoffabgabevorrichtung W, deren Aufbau aus
den Fig. 2-5 hervorgeht.

Die Vorrichtung W umfasst einen aus zwei Kunststoff-Halbschalen 11
und 12 zusammengesetzten Behälter 10, der durch eine gerippte, mit
ihrem Rand zwischen den beiden Halbschalen 11 und 12 eingeklemmte
Aluminium-Folie 13 in zwei Kammern 14 und 15 unterteilt ist. Ein
Boden 16 trennt einen sich unterhalb der beiden Kammern 14 und 15
erstreckenden Sammelraum 17 von den beiden Kammern 14 und 15 ab.
Anstatt der Aluminum-Folie kann selbstverständlich auch eine Trennwand aus Kunststoff oder einem anderen Material vorgesehen sein.

Die in der Zeichnung rechte Behälter-Schale 11 ist an ihrem oberen
Ende mit einem stutzenförmigen Einlass 18 und an ihrem unteren Ende
mit einem ebensolchen Auslass 19 versehen. Ein- und Auslass 18 bzw.
19 dienen zum Anschluss der bzw. Einfügen in die Flüssigkeitsleitung S
und sind vorzugsweise so ausgebildet (nicht dargestellt), dass die
Leitung einfach und mit wenigen Handgriffen sicher befestigt werden
kann (z.B. Bajonett-Verschluss oder dgl.).

Beide Behälterhalbschalen 11 und 12 weisen ferner in der Nähe ihres
bei vertikaler Gebrauchslage höchsten Punktes je eine Entlüftungsöffnung 21 bzw. 22 auf, die mit einem vorzugsweise hydrophoben
Filter 23 bzw. 24 versehen ist, sodass durch diese Oeffnungen bzw.
die Filter wohl Luft bzw. Gas, nicht jedoch die zu verabreichende
Flüssigkeit entweichen kann.

Abgesehen von der Aluminium-Trennwand 13 und dem Boden 16 weist der
Behälter 10 als weitere Einbauten noch vier rohrförmige Ueberläufe 25a,
25b, 26 und 27 auf, von denen sich drei in der in der Zeichnung rechten
Kammer 14 und einer in der linken Kammer 15 befinden. Einer der Ueberläufe in der rechten Kammer 14, in der Zeichnung der vordere Ueberlauf
26, und der Ueberlauf 27 in der linken Kammer 15 münden unten in den
Sammelraum 17 und damit in den Auslass 19 aus. Die Ueberläufe 25a und

0100296

- 4 -

25b münden durch Oeffnungen 28 in der Aluminium-Folie 13 in den bodennahen Bereich der linken Kammer 15.

Schliesslich sind an der Wand der linken Behälterhalbschale 12 eine
Reihe von Stützrippen 29 angeformt, welche zusammen mit der gerippten
Aluminium-Folie 13 eine hier plattenförmige Wirkstoffabgabeanordnung
30 positionieren und festhalten. Letztere ist, wie schon gesagt,
plattenförmig und besteht aus einem Zellulose-Träger, auf bzw. in
dem der zu administrierende Wirkstoff in lösbarer Form enthalten
ist. Statt des Zellulose-Trägers kann auch eine Kunststoff-Platte
mit einer Vertiefung für den dort angeordneten Wirkstoff vorgesehen
sein.

Die Funktionsweise der Vorrichtung ist wie folgt:

Die dem Patienten zu verabreichende Flüssigkeit, beispielsweise
irgendeine Infustionslösung, gelangt durch den Einlass 18 in den
Behälter 10 und durchströmt diesen nun auf zwei verschiedenen Wegen,
um ihn dann via den Auslass 19 wieder zu verlassen. Der erste Strömungsweg ist relativ direkt, nämlich vom Einlass 18 in die rechte Kammer 14
und dann über den Ueberlauf 26 und den Sammelraum 17 zum Auslass 19.
Der zweite Strömungsweg ist komplizierter: Die Flüssigkeit fliesst hier
durch die rechte Kammer 14 über die Ueberläufe 25a und 25b in die
linke Kammer 15 und von dort über den Ueberlauf 27 und den Sammelraum
17 in den Auslass 19. Der Abschnitt 28-27 des Strömungswegs vom Eintreten in die linke Kammer 15 bis zur Eintrittsöffnung des Ueberlaufs 27
wird dabei, wie aus der Zeichnung leicht erkenntlich ist, von unten
nach oben durchflossen und ist daher der allgemeinen Strömungsrichtung
der Flüssigkeit genau entgegengesetzt gerichtet. Genau in diesem verkehrt durchflossenen Abschnitt befindet sich nun die Wirkstoffabgabeanordnung 30, also der Zellulose-Träger mit dem darauf befindlichen
Wirkstoff. Die an ihm vorbeifliessende Flüssigkeit löst nun den Wirkstoff kontrolliert aus dem Träger, wobei sich dank der speziellen Anordnung des Trägers eine besonders gleichmässige und konstante Wirkstoffabgabe an die Flüssigkeit erzielen lässt.

- 5 -

Allfällig in den Kammern 14 und 15 eingeschlossene Luft kann durch
die Entlüftungsöffnungen 21 und 22 nach aussen entweichen.

Der Durchfluss und die Geschwindigkeit der Wirkstoffabgabe kann durch
entsprechende Bemessung der Ueberläufe in weiten Grenzen den jeweiligen Erfordernissen angepasst werden.

Mit dem der Wirkstoffabgabevorrichtung W nachgeschalteten Durchflussbegrenzer D, der vorzugsweise als Kapillar-Durchflussbegrenzer ausgebildet ist, kann der Durchfluss der Infusionslösung bei Bedarf
exakt und reproduzierbar eingestellt werden.

Vorstehend wurde lediglich ein besonders zweckmässiges Ausführungsbeispiel einer erfindungsgemässen Wirkstoffabgabevorrichtung
beschrieben. Es versteht sich, dass zahlreiche Abwandlungen und
Ausführungsvarianten möglich sind, die jedoch alle von der Erfindung mit umfasst sind. Wesentlich ist lediglich, dass ein entgegen
der allgemeinen Strömungsrichtung durchflossener Strömungswegabschnitt
vorhanden ist, in welchem die Wirkstoffabgabe an die Flüssigkeit erfolgt, weil durch diese umgekehrte Strömung ein gewisser Stau und
damit eine gleichmässige Strömung und damit schliesslich eine gleichmässige und konstante Wirkstoffabgabe erreicht wird. Ein weiteres
wesentliches Merkmal sind im übrigen auch die Entlüftungsöffnungen,
durch welche die im Behälter eingeschlossene Luft entweichen kann.

Die erfindungsgemässe Wirkstoffabgabevorrichtung erlaubt, wie schon
gesagt, eine sehr gleichmässige Wirkstoffabgabe und ist gleichzeitig
konstruktiv einfach, sodass sie sich bestens als Einmal-Anwendungs-
oder Wegwerfsystem eignet, wobei der Träger mit dem Wirkstoff nicht
erst bei Gebrauch eingesetzt, sondern bereits von vorneherein fertig
mit der Vorrichtung konfektioniert wird.

- 6 -

Patentansprüche
_____

1. Wirkstoffabgabevorrichtung zum Einfügen in die Flüssigkeitsleitung eines parenteralen Applikationssystems mit einem einen Einlass und einen Auslass aufweisenden, von der Flüssigkeit durchströmten Behälter und mit einer im Behälter befindlichen Anordnung zur Abgabe eines Wirkstoffs in die durchströmende Flüssigkeit, dadurch gekennzeichnet, dass im Behälter (10) zwei getrennte, je vom Einlass (18) zum Auslass (19) führende Flüssigkeitswege (18-14-26-17-19 und 18-14-25a/25b-28-15-27-17-19) ausgebildet sind, dass einer (18-14-25a, 25b-28-15-27-19) der Flüssigkeitswege einen im wesentlichen entgegen der allgemeinen Strömungsrichtung durchströmten Abschnitt (28-15-27) aufweist und dass sich die Wirkstoffabgabeanordnung (30) in diesem Abschnitt befindet.

2. Vorrichtung nach Anspruch 1, dadurch gekennzeichnet, dass der Behälter (10) zwei kommunizierende Kammern (14,15) aufweist, die beide mit einem in den Auslass (19) mündenden Ueberlauf (26,27) versehen sind, wobei der Einlass (18) in den oberen Teil der einen Kammer (14) mündet und sich die Wirkstoffabgabeanordnung (30) in der anderen Kammer (15) befindet.  ·

3. Vorrichtung nach Anspruch 2, dadurch gekennzeichnet, dass die beiden Kammern (14,15) durch einen weiteren, in der genannten einen Kammer (14) angeordneten, in den unteren Teil der genannten anderen Kammer (15) ausmündenden Ueberlauf (25a, 25b) verbunden sind.

4. Vorrichtung nach einem der vorangehenden Ansprüche, dadurch gekennzeichnet, dass der Behälter (10) aus zwei einstückigen Kunststoffschalen (11,12) besteht.

5. Vorrichtung nach Anspruch 4, dadurch gekennzeichnet, dass der Behälter (10) durch eine zwischen  den beiden Kunststoffschalen (11,12)

- 7 -

eingeklemmte Folie (13), insbesondere eine gerippte Aluminium-Folie, in zwei Kammern (14,15) unterteilt ist.

6. Vorrichtung nach einem der vorangehenden Ansprüche, dadurch gekennzeichnet, dass in seinem oberen, einlaufseitigen Teil wenigstens eine Entlüftungsöffnung (21,22) mit einem im wesentlichen nur für Gase durchlässigen Filter (23,24) angeordnet ist.

7. Vorrichtung nach einem der vorangehenden Ansprüche, dadurch gekennzeichnet, dass die Wirkstoffabgabeanordnung ein plattenförmiger Träger (30) mit darauf und/oder darin angeordnetem Wirkstoff ist.

8. Vorrichtung nach Anspruch 7, dadurch gekennzeichnet, dass im Behälter (10) Lamellen oder Rippen (29) zur Halterung und Positionierung der Wirkstoffabgabeanordnung (30) vorgesehen sind.

FO 7.7/KL/sch*

0100296

*Fig.1*

*Fig.2*

*Fig.3*

Fig.4

Fig.5

Fig.6

0100296

Nummer der Anmeldung

EP 83 81 0246

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | US-A-3 587 313 (SMITH)<br><br>--- | | A 61 M 5/14 |
| A | GB-A- 28 978 (OWENS) A.D. 1913<br><br>--- | | |
| A | US-A-2 850 211 (FERNANDEZ)<br><br>--- | | |
| A | BE-A- 648 522 (CALMIC)<br><br>--- | | |
| A | DE-A-2 550 340 (GRIMSRUD)<br><br>----- | | |
| | | | RECHERCHIERTE SACHGEBIETE (Int. Cl. ³) |
| | | | A 61 M |

Der vorliegende Recherchenbericht wurde für alle Patentansprüche erstellt.

| Recherchenort<br>DEN HAAG | Abschlußdatum der Recherche<br>30-09-1983 | Prüfer<br>VANRUNXT J.M.A. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82